# EUROPÄISCHE PATENTANMELDUNG

(11) **EP 0 573 391 A1**
(43) Veröffentlichungstag der Anmeldung: **08.12.1993**
(21) Anmeldenummer: 93810381.9
(22) Anmeldetag: 25.05.1993
(51) Int. Cl.: C07D 277/54, A61K 31/425, C08G 77/50

(54) **Thiosemicarbazonsäureester und ihre Anwendung als Arzneimittelwirkstoff**

(30) Priorität: 03.06.1992 CH 1777/92
(71) Anmelder: CIBA-GEIGY AG, CH-4002 Basel (CH)
(72) Erfinder: Missbach, Martin, Dr., CH-4310 Rheinfelden (CH)

(57) **Zusammenfassung**

Die Erfindung betrifft neue Thiosemicarbazonsäureester der Formel I 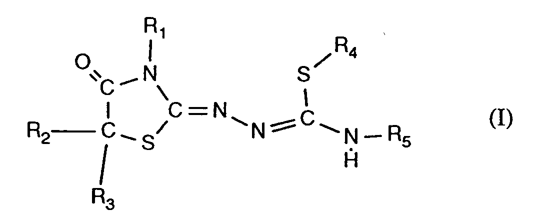
worin R₁ und R₅ Niederalkyl, Niederalk-2-en-1-yl oder auch Niederalk-2-in-1-yl bedeuten,
R₂ und R₃ unabhängig voneinander Wasserstoff oder Niederalkyl bedeuten oder gemeinsam für Niederalkyliden stehen und
R₄ Niederalkyl, Niederalkoxy, Niederalk-2-en-1-yl, Niederalk-2-in-1-yl, Arylniederalkyl, Arylniederalkenyl, oder auch Niederalkoxycarbonylniederalkyl bedeutet und ihre Salze und tautomeren Verbindungen und Doppelbindungsisomeren, Verfahren zur Herstellung der genannten Verbindungen, diese enthaltende pharmazeutische Präparate und ihre Verwendung als Arzneimittelwirkstoffe.

## Beschreibung

Die Erfindung betrifft neue Thiosemicarbazonsäureester der Formel I 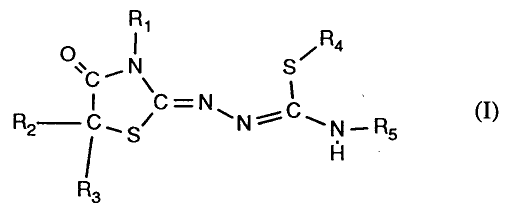
worin R₁ und R₅ Niederalkyl, Niederalk-2-en-1-yl oder auch Niederalk-2-in-1-yl bedeuten,
R₂ und R₃ unabhängig voneinander Wasserstoff oder Niederalkyl bedeuten oder gemeinsam für Niederalkyliden stehen und
R₄ Niederalkyl, Niederalkoxy, Niederalk-2-en-1-yl, Niederalk-2-in-1-yl, Arylniederalkyl, Arylniederalkenyl, oder auch Niederalkoxycarbonylniederalkyl bedeutet und ihre Salze und tautomeren Verbindungen und Doppelbindungsisomeren, Verfahren zur Herstellung der genannten Verbindungen, diese enthaltende pharmazeutische Präparate und ihre Verwendung als Arzneimittelwirkstoffe.

Vor- und nachstehend sind unter niederen Resten und Verbindungen beispielsweise solche zu verstehen, die bis und mit 7, vorzugsweise bis und mit 4, Kohlenstoffatome (C-Atome) aufweisen.

Niederalk-2-en-1-yl ist beispielsweise C₃-C₅-Alk-2-en-1-yl, wie insbesondere Allyl, Methallyl, oder Dimethylallyl.

Niederalk-2-in-1-yl ist beispielsweise C₃- C₅-Alk-2-in-1-yl, wie insbesondere Prop-2-in-1-yl oder auch But-2-in-1-yl
Niederalykl ist beispielsweise C₁- C₄-Alkyl, wie Methyl, Ethyl, Propyl oder Butyl.

Niederalkyliden ist beispielsweise C₁-C₄-Alkyliden, wie insbesondere Methylen oder Ethyliden.

Niederalkoxy ist z.B. n-Propoxy, Isopropoxy, n-Butoxy oder tert.-Butoxy, vorzugsweise Ethoxy, und vor allem Methoxy.

Aryl - in zusammengesetzten Begriffen wie z.B. Arylniederalkyl oder Arylniederalkenyl ist z.B. Phenyl oder Naphthyl, wie 1- oder 2-Naphthyl, oder substituiertes Phenyl oder Naphthyl, wie z.B. Phenyl oder Naphthyl, welche durch Niederalkyl, Halogen-niederalkyl, Hydroxy, Niederalkoxy, Niederalkanoyloxy, Halogen, Cyano und/oder Nitrosubstituiert sind. Aryl ist bevorzugt unsubstituiert oder wie oben angegeben substituiertes Phenyl, und insbesondere Phenyl.

Arylniederalkyl ist vorzugsweise Phenylniederalkyl und insbesondere Benzyl.

Arylniederalkenyl ist vorzugsweise Phenylniederalkenyl, insbesondere Phenylallyl oder auch Phenylvinyl (Phenylethylen).

Niederalkoxcarbonylniederalkyl ist z.B. Propoxycarbonyl- oder Butoxycarbonyl-, vorzugsweise Methoxycarbonyl- und insbesondere Ethoxycarbonyl-niederalkyl, wie z.B. Methoxycarbonyl- oder Ethoxycarbonyl-ethyl oder -methyl.

Pharmazeutisch verwendbare Säureadditionssalze von Verbindungen der Formel I sind beispielsweise deren pharmazeutisch verwendbaren Salze mit geeigneten Mineralsäuren, wie Halogenwasserstoffsäuren, Schwefelsäure oder Phosphorsäure, z.B. Hydrochloride, Hydrobromide, Sulfate, Hydrogensulfate oder Phosphate, Salze mit geeigneten aliphatischen oder aromatischen Sulfonsäuren oder N-substituierten Sulfaminsäuren, z.B. Methansulfonaten, Benzolsulfonate, p-Toluolsulfonate oder N-Cyclohexylsulfaminate(Cyclamate), oder Salze mit starken organischen Carbonsäuren, wie Niederalkancarbonsäuren oder gegebenenfalls ungesättigten oder hydroxylierten aliphatischen Dicarbonsäuren, z.B. Acetate, Oxalate, Malonate, Maleinate, Fumarate, Maleate, Tatrate oder Citronate, Salze von Verbindungen der Formel I sind beispielsweise deren Säureadditionssalze, beispielsweise deren pharmazeutisch verwendbaren Salze mit geeigneten Mineralsäuren, wie Halogenwasserstoffsäuren, Schwefelsäuren oder Phosphorsäure, z.B. Hydrochloride, Hydrobromide, Sulfate, Hydrogensulfate oder Phosphate, oder Salze mit geeigneten aliphatischen oder aromatischen Sulfonsäuren oder N-substituierten Sulfaminsäuren, z.B. Methansulfonate, Benzolsulfonate, p-Toluolsulfonate oder N-Cyclohexylsulfaminate Cyclamate).

Die Verbindungen der Formel I und ihre pharmazeutisch verwendbaren Salze weisen wertvolle pharmakologische Eigenschaften auf. Diese können in vivo beispielsweise am Modell der Adjuvans-Arthritis der Ratte nach I. Wiesenberg et al. Clin. Exp. Immunol. 78, 245 (1989) im Dosisbereich ab etwa 0,1 bis etwa 10,0 mg/kg p.o. oder i.p. insbesondere ab etwa 0,1 bis etwa 3,0 mg/kg p.o. oder i.p. nachgewiesen werden.

Die Verbindungen der Formel I und ihre pharmazeutisch verwendbaren Salze können deshalb zur Behandlung von Erkrankungen des rheumatoiden Formenkreises eingesetzt werden. Zu diesen gehören insbesondere die rheumatoide Arthritis, die ankylosierende Spondylitis und andere seronegative Spondylathritiden, z.B. Spondylarthritis bei Colitis ulcerosa und Morbus Crohn, aber auch reaktive Arthritiden, Kollagenkrankheiten wie Lupus erythematosus, degenerative rheumatische Erkrankungen, extraartikuläre rheumatische und pararheumatische Erkrankungen, z.B. Gicht und Osteoporose.

Die Erfindung betrifft in erster Linie Verbindungen der Formel I, worin R₁ und R₅ C₁-C₄-Alkyl, C₃-C₅-Alk-2-en-1-yl oder C₃-C₅-Alk-2-in-1-yl bedeuten, R₂ und R₃ unabhängig voneinander Wasserstoff oder C₁-C₄-Alkyl bedeuten oder gemeinsam C₁-C₄-Alkyliden darstellen und R₄ C₁-C₄-Alkyl, C₁-C₂-Alkoxy, C₃-C₅-Alk-2-en-1-yl, C₃-C₅-Alk-2-in-1-yl, Phenyl-C₁-C₂-alkyl bedeutet, und ihre Salze und tautomeren Verbindungen und Doppelbindungsisomeren.

Die Erfindung betrifft vor allem Verbindungen der Formel I, worin der Rest R₁ C₃-C₅-Alk-2-en-1-yl, wie Allyl oder Methallyl oder C₃-C₅-Alk-2-in-1-yl, wie Prop-2-in-1-yl bedeutet, R₂ und R₃ beide Wasserstoff oder gleiche C₁-C₄-Alkylgruppen, wie Methylgruppen bedeuten oder gemeinsam C₁-C₄-Alkyliden, wie Methylen darstellen. R₄ C₁-C₄-Alkyl, wie Methyl oder Ethyl, C₁-C₂-Alkoxy, wie Methoxy oder Ethoxy C₃-C₅-Alk-2-en-1-yl, wie Allyl, Methallyl oder Dimethylallyl, C₃-C₅-Alk-2-in-1-yl, wie Prop-2-in-1-yl oder Phenyl-C₁-C₂-alkyl, wie Benzyl bedeutet und R₅ C₃-C₅-Alk-2-en-1yl, wie Allyl oder Methallyl oder C₁-C₄-Alkyl, wie Methyl oder Ethyl darstellt und ihre Salze,insbesondere ihre pharmazeutisch verwendbaren Salze und tautomeren Verbindungen und Doppelbindungsisomeren.

Die Erfindung betrifft insbesondere Verbindungen der Formel I, worin R₁ Allyl, Methallyl oder Prop-2-in- 1-yl bedeutet, R₂ und R₃ beide Wasserstoff oder Methyl bedeuten und R₄ Allyl, Dimethylallyl, Prop-2-in-1-yl oder Benzyl darstellt und R₅ die Bedeutung von Allyl oder Methyl hat und ihre Salze, insbesondere ihre pharmazeutisch verwenbaren Salze und tautomeren Verbindungen und Doppelbindungsisomeren.

Die Erfindung betrifft namentlich die in den Beispielen genannten Verbindungen der Formel I und ihre Salze, insbesondere ihre pharmazeutisch verwendbaren Salze.

Die neuen Verbindungen der Formel I können in an sich bekannter Weise hergestellt werden, in dem man
eine Verbindung der Formel II 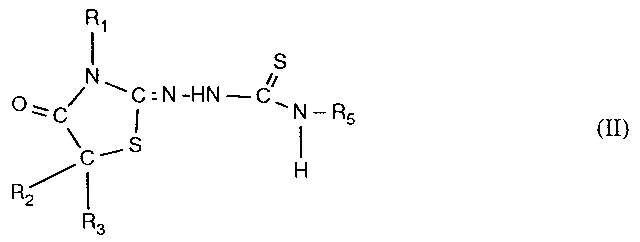
mit einer Verbindung der Formel III

R₄-X (III)

in der X eine nukleofuge Abgangsgruppe ist und R₁,R₂,R₃,R₄ und R₅ die oben angegebenen Bedeutungen haben, umsetzt.

Der Rest X in einer Verbindung der Formel III ist bevorzugt Halogen, z.B. Chlor, Brom oder Jod, ferner aber auch z.B. aliphatisch oder aromatisch-substituiertes Sulfonyloxy, z.B. Methylsulfonyloxy (Mesilat) oder 4-Methylphenylsulfonyloxy (Tosylat). Als weitere Abgangsgruppe X kann auch z.B. die Trifluoracetatgruppe in einer Verbindung der Formel III R₄-X verwendet werden.

Die Kondensation einer Verbindung der Formel II mit einer Verbindung der Formel III erfolgt in Gegenwart eines basischen Kondensationsmittels, wie zum Beispiel einer tertiären organischen Base, wie Triniederalkylamin, beispielsweise Triethylamin, von Hünig'scher Base oder einer organischen Stickstoffbase, wie Pyridin oder Chinolin in einem Temperaturbereich von 25 - 120°C, vorteilhaft bei Siedetemperatur des Lösungsmittels.

Als Lösungsmittel können protische und aprotische Lösungsmittel, wie z.B. aliphatische Halogenkohlenwasserstoffe, beispielsweise Dichlormethan, insbesondere Methylenchlorid oder aliphatische oder cycloaliphatische Ether wie z.B. Tetrahydrofuran oder Dioxan verwendet werden.Ferner können Toluol oder auch Ethanol als Lösungsmitel verwendet werden.

Gegebenenfalls können auch Salze wie z.B. Natrium- oder Kaliumjodid und/oder katalytische Mengen von Dimethylaminopyridin als Reaktionsbeschleuniger hinzugesetzt werden.

Die Verbindungen der Formel II und auf an sich bekannte Methoden beruhende Verfahren zu ihrer Herstellung sind bekannt und beispielsweise in GB-1,325,061, US-4,697,020, DE-2,405395 oder DE-2,632,745 und als Zwischenprodukte für die Herstellung von tumorhemmenden Arzneimittelwirkstoffen beschrieben.

Verfahrensgemäss erhältliche Verbindungen können in üblicher Weise in andere Verbindungen der Formel I überführt werden.

Erhaltene Salze können in an sich bekannter Weise in die freien Verbindungen umgewandelt werden, z.B. durch Behandeln mit einer Base, wie einem Alkalimetallhydroxid, einem Metallcarbonat oder -hydrogencarbonat, oder Ammoniak, oder einer anderen eingangs genannten salzbildenden Base bzw. mit einer Säure, wie einer Mineralsäure, z.B. mir Chlorwasserstoff, oder einer anderen eingangs genannten salzbildenden Säure.

Erhaltene Salze können in an sich bekannter Weise in andere Salze überführt werden, Säureadditionssalze z.B. durch Behandeln mit einem geeigneten Metallsalz, wie einem Natrium-, Barium- oder Silbersalz, einer anderen Säure in einem geeigneten Lösungsmittel, in welchem ein sich bildendes anorganisches Salz unlöslich ist und damit aus dem Reaktionsgleichgewicht ausscheidet,und Basesalze durch Freisetzung der freien Säure und erneute Versalzung.

Die Verbindungen der Formel I, einschliesslich ihrer Salze, können auch in Form von Hydraten erhalten werden oder das zur Kristallisation verwendete Lösungsmittel einschliesslich.

Infolge der engen Beziehung zwischen den neuen Verbindungen in freier Form und in Form ihrer Salze sind vorstehend und nachfolgend unter den freien Verbindungen und ihren Salze sinn- und zweckgemäss gegebenenfalls auch die entsprechenden Salze bzw. freien Verbindungen zu verstehen.

Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Umsetzung des erhaltenen Diastereomerengemisches bzw. Racemates mit einer optisch aktiven Hilfsverbindung, z.B. entsprechend der in Verbindungen der Formel I enthaltenen sauren, basischen oder einem optisch aktiven Alkohol, in Gemische diastereomerer Salze bzw. funktioneller Derivate, wie Ester, Trennung derselben in die Diastereomeren, aus denen das jeweils gewünschte Enatiomere in der jeweils üblichen Weise freigesetzt werden kann. Dafür geignete Basen, Säuren bzw, Alkohole sind beispielsweise optisch aktive Alkaloidbasen, wie Strychnin, Cinchonin oder Brucin, oder D- oder L-(l-Phenyl)ethylamin, 3-Pipecolin, Ephedrin, Amphetamin und ähnliche synthetisch zugängliche Basen, optisch aktive Carbon- oder Sulfonsäuren, wie Chinasäure oder D- oder L-Weinsäure, D- oder L-Di-o-toluylweinsäure, bzw. optisch aktive Alkohole, wie Borneol oder D- oderL-(1-Phenyl)ethanol.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes verwendet oder insbesondere unter den Reaktionsbedingungen bildet.

Die neuen Ausgangsstoffe, die speziell für die Herstellung der erfindungsgemässen Verbindungen entwickelt wurden, insbesondere die zu den eingangs als bevorzugt gekennzeichneten Verbindungen der Formel I führende Ausgangsstoffauswahl, die Verfahren zu ihrer Herstellung und ihre Verwendung als Zwischenprodukte bilden ebenfalls einen Gegenstand der Erfindung.

Bei den erfindungsgemässen pharmazeutischen Präparaten, welche die erfindungsgemässe Verbindung oder pharmazeutisch verwendbare Salze davon enthalten, handelt es sich um solche zur enteralen, wie oralen, ferner rektalen, und parenteralen Verabreichung an Warmblüter(n), wobei der pharmakologische Wirstoff allein oder zusammnen mit einem pharmazeutisch anwendbaren Trägermaterial enthalten ist. Die tägliche Dosierung des Wirkstoffes hangt von dem Alter und dem individuellen Zustand sowie von der Applikationsweise ab.

Die neuen pharmazeutischen Präparate enthalten z.B. von etwa 10% bis etwa 80%, vorzugsweise von etwa 20% bis etwa 60%, des Wirkstoffs. Erfindungsgemässe pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfstoffen zu Tabletten oder Dragée-Kernen verarbeitet.

Geeignete Trägerstoffe sind insbesondere Füllstoffe, wie Zucker,z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Tragakanth, Methylcellulose und/oder Polyvinylpyrrolidon, wenn erwünscht, Sprengmittel,wie die oben genannten Stärken, ferner Craboxymethylstärke, quervernetztes Polyvinylpyrrolidon,Agar, Alginsäure oder ein Salz davon, wie Natriumalginat. Hilfsmittel sind in erster Linie Fliess-, Fliessregulier- und Schmiermittel, z.B. Kieselsäure, Talk, Stearinsäure oder Salze davon, wie Magnesium- oder Calciumstearat, und/oder Polyethylenglykol. Dragée-Kerne werden mit geeigneten, gegebenenfalls Magensaft-resistenten Ueberzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelngemische oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulates, z.B. im Gemisch mit Füllstoffen, wie Lactose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Oelen, Paraffinöl oder flüssigen Polyethylenglykol, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykol oder höhere Alkanole. Ferner können auch Gelatine-Rektalkapseln verwendet werden, die eine Kombination des Wirkstoffs mit einem Grundmassenstoff enthalten. Als Grundmassenstoff kommen z.B. flüssige Triglyceride, Polethylenglykol oder Paraffinkohlenwasserstoffe in Frage.

Zur parenteralen Verabreichung eignen sich in erster Linie wässrige Lösungen eines Wirkstoff in wasserlöslicher Form, z.B. eines wasserlöslichen Salzes, ferner Suspensionen des Wirkstoffs, wie entsprechende ölige Injektionssuspension, wobei man geeignete lipophile Lösungsmittel oder Vehikel, wie fette Oele,z.B. Sesamöl,oder synthetische Fettsäureester, z.B. Ethyloleat oder Triglyceride, verwendet oder wässrige Injektionssuspensionen, welche viskositätserhöhende Stoffe, z.B. Natrium-carboxymethylcellulose, Sorbit und/oder Dextran, und gegebenenfalls auch Stabilisatoren enthalten.

Die Erfindung betrifft ebenfalls die Verwendung der Verbindung der Formel I, vorzugsweise in Form von pharmazeutischen Präparaten. Die Dosierung des Wirkstoffes hängt von der Warmblüter-Spezies, dem Alter und dem individuellen Zustand sowie der Applikationsweise ab. Im Normfall ist für einen etwa 75 kg schweren Patienten bei oraler Applikation eine ungefahre Tagesdosis von etwa 5 mg bis etwa 1000 mg, insbesondere von etwa 10 mg bis 200 mg zu veranschlagen. Diese kann auf eimal gegeben werden oder auf mehrere, z.B. 2 bis 4 Einzeldosen verteilt werden. Pharmazeutische Präparate in Dosiseinheitsform enthalten somit von etwa 5 mg bis etwa 250 mg, insbesondere von etwa 10 mg bis 50 mg Wirkstoff.

Die nachfolgenden Beispiele dienen zur Illustration der Erfindung; Temperaturen sind in Celsiusgraden, Drucke in mbar angegeben.

### Beispiel 1:

Bei Raumtemperatur werden zu 2,5 g 1-(3-Allyl-4-oxo-thiazolidin-2-yliden)-4-methyl-thiosemicarbazon in 30 ml Methylenchlorid 1,5 g Allylbromid unter Rühren zugegeben und das erhaltene Gemisch wird bis zum Sieden des Lösungsmittels erwärmt und für 4 Stunden unter Rückfluss gehalten. Nach dem Abkühlen auf Raumtemperatur wird das Lösungsmittel am Rotationsverdampfer abgezogen und der erhaltene Rückstand wird in Methylenchlorid aufgenommen, mit gesättigter Natriumhydrogencarbonatlösung gewaschen, über Magnesiumsulfat getrocknet, eingeengt und nach Kristallisation aus Ether/Petrolether und weiterer Umkristallisation aus Ethanol erhält man den reinen 1-(3-Allyl-4-oxo-thiazolidin-2-yliden)-4-methyl-thiosemicarbazonsäure-allylester mit einem F.P. 117 -118°C.

### Beispiel 2:

Bei Raumtemperatur werden zu 1,2 g 1-(3-Allyl-4-oxo-thiazolidin-2-yliden)-4-methyl-thiosemicarbazon in 30 ml Tetrahydrofuran 0,75 g Dimethylallylbromid in Gegenwart von 0,5 g Triethylamin und einer Spatelspitze Dimethylaminopyridin unter Rühren zugegeben und das erhaltene Gemisch wird bis zum Sieden des Lösungsmittels erwärmt und für 2 Stunden unter Rückfluss gehalten. Nach dem Abkühlen auf Raumtemperatur wird das Lösungsmittel am Rotationsverdampfer abgezogen und der erhaltene Rückstand in Methylenchlorid aufgenommen, mit Wasser gewaschen, über Magnesiumsulfat getrocknet, eingeengt und nach Kristallisation aus Methylenchlorid/Ether werden die Kristalle in Aceton gelöst und mit 1 ml 6N HCl in Ethanol versetzt. Nach Filtration und Trocknen bei Raumtemperatur am Hochvakuum wird das 1-(3-Allyl-4-oxo-thiazolidin-2-yliden)-4-methyl-thiosemicarbazonsäure-dimethylallylester-hydrochlorid mit einem F.P. 119 - 121°C erhalten.

### Beispiel 3:

Analog zu Beispiel 1 werden zu 2,5 g 1-(5,5-Dimethyl-3-methallyl-4-oxo-thiazolidin-2-yliden)-4-allyl-thiosemicarbazon in 20 ml Methylenchlorid 1,2 g Allylbromid hinzugegeben und anschliessend 12 Stunden lang unter Rückfluss erwärmt. Nach Aufarbeitung wie oben unter Beispiel 1 beschrieben, wird das farblose Oel in Ethanol gelöst, mit 1,5 ml 6N HCl versetzt und Ether zugegeben. Nach Filtration erhält man farblose Kristalle des 1-(5,5-Dimethyl-3-methallyl-4-oxo--thiazolidin-2-yliden)-4-allyl-thiosemicarbazonsäure-allylester-hydrochlorids mit einem F.P. von 127-129°C.

### Beispiel 4:

Analog zu Beispiel 2 werden 1,6 g 1-(5,5-Dimethyl-3-methallyl-4-oxo-thiazolidin-2-yliden)-4-allyl-thiosemicarbazon in 25 ml Methylenchlorid und 0.7 g Methyljodid in Gegenwart von 0,5 g Triethylamin 24 Stunden bei Raumtemperatur gerührt.

Anschliessend werden nochmals 0,5 g Methyljodid und 0,3 g Triethylamin zugegeben und für 5 Stunden am Rückfluss gekocht. Nach Aufarbeitung analog zu Beispiel 2 und Chromatographie an Kieselgel mit Methylenchlorid und Kristallisation aus Ether/Petrolether erhält man das 1-(5,5-Dimethyl-3-methallyl-4-oxo-thiazolidin-2-yliden)-4-allylthiosemicarbazonsäure-allylester-hydrochlorid in einem Diastereomerenverhaltnis von 5:1 mit einem F.P. 98 -104°C.

### Beispiel 5:

Analog zu Beispiel 2 werden zu 1,2 g 1-(3-Allyl-4-oxo-thiazolidin-2-yliden)-4-methyl-thiosemicarbazon in 15 ml Methylenchlorid 0,8 g Methyljodid in Gegenwart von 1,4 ml Triethylamin 24 Stunden lang unter Rückfluss erwärmt. Nach oben, im Beispiel 2 beschriebener Aufarbeitung und Chromatographie an Kieselgel wird der 1-(3-Allyl-4-oxo-thiazolidin-2-yliden)-4-methyl-thiosemicarbazonsäuremethylester in einem Diastereomerenverhältnis von 3:1 erhalten, F.P. 127 -128°C.

### Beispiel 6:

Analog zu Beispiel 2 werden 1.2 g 1-(3-Allyl-4-oxothiazolidin-2-yliden)-4-methyl-thiosemicarbazon in 30 ml Tetrahydrofuran und 0.9 g Benzylbromid in Gegenwart von 0.5 g Triethylamin für 5 Stunden am Rückfluss gekocht. Nach Aufarbeitung und Kristallisation aus Methylenchlorid/Ether werden die Kristalle in Aceton gelöst und mit 1 ml 6N HCl in Ethanol versetzt, woraus Kristalle des 1-(3-Allyl-4-oxo-thiazolidin-2-yliden)-4-methyl-thiosemicarbazonsäurebenzylester-hydrochlorids durch Filtration im Diastereomerenverhältnis von 7:1 mit einem F.P. 139 - 141°C erhalten werden.

### Beispiel 7:

Analog zu Beispiel 2 werden 1,2 g 1-(3-Allyl-4-oxo-thiazolidin-2-yliden)-4-methyl-thiosemicarbazon in 30 ml Ethanol und 1,1 g 2-Brommethyl-phenylether in Gegenwart von 0,5 g Triethylamin und 0,3 g Natriumjodid für 24 Stunden am Rückfluss gekocht. Nach Aufarbeitung und Chromatographie an Kieselgel mit Methylenchlorid und Kristallisation aus Ether erhält man Kristalle des 1-(3-Allyl-4-oxo-thiazolidin-2-yliden)-4-methyl-thiosemicarbazonsäure-2-phenoxyethylesters mit einem F.P. 106-107°C.

### Beispiel 8:

Analog zu Beispiel 2 werden 1,2 g 1-(3-Allyl-4-oxo-thiazolidin-2-yliden)-4-methylthiosemicarbazon in 30 ml Tetrahydrofuran und 1,0 g 3-Phenyl-allyl-1-bromid in Gegenwart von 0,5 g Triethylamin und einer Spatelspitze Dimethylaminopyridin für 10 Stunden unter Rückfluss erwärmt. Nach Aufarbeitung und Kristallisation aus Methylenchlorid/Ether werden die Kristalle in Aceton gelöst und mit 1 ml 6N HCl in Ethanol versetzt, woraus nach Zugabe von Ether farblose Kritalle des 1-(3-Allyl-4-oxo-thiazolidin-2-yliden)-4-methyl-thiosemicarbazonsäure-styrolester-hydrochlorids erhalten werden, F.P. 107-110°C.

### Beispiel 9:

Analog zu Beispiel 2 werden 2,4 g 1-(3-Allyl-4-oxo-thiazolidin-2-yliden)-4-methyl-thiosemicarbazon in 30 ml Ethanol und 3,2 g 3-(2,4-Dihydroxy-3-propyl-acetophenon-4-yl)-propylbromid in Gegenwart von 2,8 ml Triethylamin 0,2 g Natriumjodid für 5 Stunden am Rückfluss gekocht. Nach Aufarbeitung und einfacher Umkristallisation aus Ethanol werden Kristalle des 1-(3-Allyl-4-oxo-thiazolidin-2-yliden)-4-methyl-thiosemicarbazonsäure-3-(2,4-dihydroxy- 3-propyl-acetophenon-4-yl)-propylesters erhalten, F.P. 108-110°C.

### Beispiel 10:

Analog zu Beispiel 2 werden 2 g 1-(3-Allyl-4-oxo-thiazolidin-2-yliden)-4-methyl-thiosemicarbazon und 0,7 ml Bromethanol in 30 ml Ethanol in Gegenwart von 1,4 ml Triethylamin, einer katalytischen Menge Dimethylaminopyridin und 0,3 g Natriumjodid für 15 Stunden am Rückfluss gekocht. Nach Aufarbeitung und Chromatographie an Kieselgel mit Essigester und Kristallisation aus Ether werden Kristalle des 1-(3-Allyl-4-oxo-thiazolidin-2-yliden)-4-methyl-thiosemicarbazonsäure-2-hydroxy- ethylesters in einem Diastereomerenverhältnis von 10:1 mit einem F.P. 103-105°C.

### Beispiel 11:

Analog zu Beispiel 2 werden 1.2 g 1-(3-Allyl-4-oxo-thiazolidin-2-ylidin)-4-methyl-thiosemicarbazon und 1.0 g 4-Fluorobenzylbromid in 20 ml Tetrahydrofuran in Gegenwart von 0.5 g Triethylamin und einer Spatelspitze Dimethylaminopyridin für 4 Stunden am Rückfluss gekocht. Nach Aufarbeitung und Chromatographie an Kieselgel mit Methylenchlorid und Kristallisation aus Methylenchlorid/Ether werden Kristalle des 1-(3-Allyl-4-oxo-thiazolidin-2-yliden)-4-methyl-thiosemicarbazonsäure-4-fluorobenzylester in einem Diastereomerenverhältnis von 6:1 erhalten werden, F.P. 105-106°C.

### Beispiel 12:

Analog zu Beispiel 2 werden 1.2 g 1-(3-Allyl-4-oxo-thiazolidin-2-yliden)-4-methyl-thiosemicarbazon und 1.1 g 2-Brommethylbenzonitril in 20 ml Tetrahydrofuran in Gegenwart von 0.5 g Triethylamin und einer Spatelspitze Dimethylaminopyridin für 5 Stunden am Rückfluss gekocht. Nach Aufarbeitung und Kristallisation aus Methylenchlorid/Ether werden farblose Kristalle des 1-(3-Allyl-4-oxo-thiazolidin-2-yliden)-4-methyl-thiosemicarbazon- säure-2-cyanobenzylester in einem Diastereomerenverhältnis von 4:1 erhalten, F.P. 151-152°C.

### Beispiel 13:

Analog zu Beispiel 2 werden 1.2 g 1-(3-Allyl-4-oxo-thiazolidin-2-yliden)-4-methyl-thiosemicarbazon und 0.7 g Propargylbromid in 20 ml Tetrahydrofuran in Gegenwart von 0.5 g Triethylamin und einer Spatelspitze Dimethylaminopyridin für 4 Stunden am Rückfluss gekocht. Nach Aufarbeitung und Chromatographie an Kieselgel mit Methylenchlorid werden nach Kristallisation aus Methylenchlorid/Ether farblose Kristalle von 1-(3-Allyl-4-oxo-thiazolidin-2-yliden)-4-methyl-thiosemicarbazonsäure-1-propargylester im Diastereomerenverhältnis von 3:1 erhalten, F.P. 91-92°C.

### Beispiel 14:

Tabletten, enthaltend je 10 mg 1-(3-Allyl-4-oxo-thiazolidin-2-yliden)-4-methyl-thio-semicarbazonsäure-allylester oder ein Salz davon, können wie folgt hergestellt werden:

### Zusammensetzung (10000 Tabletten)

- Wirkstoff: 100,0 g
- Lactose: 450,0 g
- Kartoffelstärke: 350,0 g
- Gelatine: 10,0 g
- Talk: 60,0 g
- Magnesiumstearat: 10,0 g
- Siliciumdioxid (hochdispers): 20,0 g
- Ethanol: q.s.

Der Wirkstoff wird mit der Lactose und 292 g Kartoffelstärke vermischt, die Mischung mit einer ethanolischen Lösung der Gelatine befeuchtet und durch ein Sieb granuliert. Nach dem Trocknen mischt man den Rest der Kartoffelstärke, das Magnesiumstearat, das Talk und das Siliciumdioxid zu und presst das Gemisch zu Tabletten von je 100,0 mg Gewicht und 10,0 mg Wirkstoffgehalt, die gewünschtenfalls mit Teilkerben zur feineren Anpassung der Dosierung versehen sein können.

### Beispiel 15:

Gelatinesteckkapseln, enthaltend 20 mg Wirkstoff, 1-(3-Allyl-4-oxo-thiazolidin-2-yliden)-4-methyl-thiosemicarbazonsäure-allylester der ein Salz davon, können z.B. folgendermassen hergestellt werden:

### Zusammensetzung (für 1000 Kapseln)

- Wirkstoff: 20,0 g
- Lactose: 240,0 g
- mikrokristalline Zellulose: 30,0 g
- Natriumlaurylsulfat: 2,0 g
- Magnesiumstearat: 8,0 g

Das Natriumlaurylsulfat wird durch ein Sieb mit einer Maschenweite von 0,2 mm zu dem lyophilisierten Wirkstoff hinzugesiebt. Beide Komponenten werden innig vermischt. Dann wird zunächst die Lactose durch ein Sieb mit einer Maschenweite von 0,6 mm und dann die mikrokristalline Zellulose durch ein Sieb mit einer Maschenweite von 0,9 mm hinzugesiebt. Daraufhin wird erneut 10 Minuten innig gemischt. Zuletzt wird das Magnesiumstearat durch ein Sieb mit einer Maschenweite von 0,8 mm hinzugesiebt. Nach 3- minütigem weiteren Mischen werden je 300 mg der erhaltenen Formulierung in Gelatinesteckkapseln der Grösse 0 abgefüllt.

### Beispiel 16:

Gelatinesteckkapseln, enthaltend 100 mg Wirkstoff, z.B. 1-(3-Allyl-4-oxo-thiazolidin-2-yliden)-4-methyl-thiosemicarbazonsäure-allylester oder ein Salz davon, können z.B. folgendermassen hergestellt werden:

### Zusammensetzung (für 1000 Kapseln)

- Wirkstoff: 100,0 g
- Lactose: 250,0 g
- mikrokristalline Zellulose: 30,0 g
- Natriumlaurylsulfat: 2,0 g
- Magnesiumstearat: 8,0 g

Das Natriumlaurylsulfat wird durch ein Sieb mit einer Maschenweite von 0,2 mm zu dem lyophilisierten Wirkstoff hinzugesiebt. Beide Komponenten werden innig vermischt. Dann wird zunächst die Lactose durch ein Sieb mit einer Maschenweite von 0,6 mm und dann die mikrokristalline Zellulose durch ein Sieb mit einer Maschenweite von 0,9 mm hinzugesiebt. Daraufhin wird erneut 10 Minuten innig gemischt. Zuletzt wird das Magnesiumstearat durch ein Sieb mit einer Maschenweite von 0,8 mm hinzugesiebt. Nach 3- minütigem weiteren Mischen werden je 390 mg der erhaltenen Formulierung in Gelatinesteckkapseln der Grösse 0 abgefüllt.

### Beispiel 17:

Lacktabletten, enthaltend je 50 mg 1-(3-Allyl-4-oxo-thiazolidin-2-yliden)-4-methyl-thiosemicarbazonsäure-allylester oder ein Salz davon, können wie folgt hergestellt werden:

### Zusammensetzung (für 1000 Lacktabletten)

- Wirkstoff: 50,0 g
- Lactose: 100,0 g
- Maisstärke: 70,0 g
- Talk: 10,0 g
- Calciumstearat: 2,0 g
- Hydroxypropylmethylcellulose: 2,36 g
- Schellack: 0,64 g
- Wasser: q.s.
- Methylenchlorid: q.s.

Der Wirkstoff, die Lactose und 40 g der Maisstärke werden gemischt und mit einem Kleister, hergestellt aus 15 g Maisstärke und Wasser (unter Erwärmen), befeuchtet und granuliert. Das Granulat wird getrocknet, der Rest der Maisstärke, der Talk und das Calciumstearat zugegeben und mit dem Granulat vermischt. Das Gemisch wird zu Tabletten (Gewicht: 240 mg) verpresst und diese mit einer Lösung der Hydroxypropylmethylcellulose und des Schellacks in Methylenchlorid lackiert; Endgewicht der Lacktablette: 283 mg.

### Beispiel 18:

Eine 0,2%-ige Injektions- oder Infusionslösung von 1-(3-Allyl-4-oxo-thiazolidin-2-yliden)-4-methyl-thiosemicarbazonsäure-allylester oder ein Salz davon, kann beispielsweise folgendermassen hergestellt werden:

### Zusammensetzung (für 1000 Ampullen)

- Wirkstoff: 5.0 g
- Natriumchlorid: 22,5 g
- Phosphatpuffer pH= 7,4: 300,0 g
- entmineralisiertes Wasser: ad 2500,0 ml

Der Wirkstoff und das Natriumchlorid werden in 1000 ml Wasser gelöst und durch ein Mikrofilter filtriert. Man versetzt mit der Pufferlösung und füllt mit Wasser auf 2500 ml auf. Zur Herstellung von Dosiseinheitsformen werden je 1,0 oder 2,5 ml in Glasampullen abgefüllt, die dann je 2,0 bzw. 5,0 mg Wirkstoff enthalten.

### Beispiel 19:

1%-ige Salbe (O/W-Emulsion), als Wirkstoff z.B 1-(3-Allyl-4-oxo-thiazolidin-2-yliden)-4-methyl-thiosemicarbazonsäure-allylester oder ein Salz davon, enthaltend, mit folgender Zusammensetzung:
- Wirkstoff: 1,0 g
- Cetylalkohol: 3,0 g
- Glycerin: 6,0 g
- Methylparaben: 0,18 g
- Propylparaben: 0,05 g
- Arlacel 60: 0,6 g
- Tween 60: 4,4 g
- Stearinsäure: 9,0 g
- Isopropylpalmitat: 2,0 g
- Paraffinöl dickflüssig: 10,0 g
- Wasser entmin. q.s. ad: 100,0 g

### Beispiel 20:

1%-iges Gel, als Wirkstoff z.B. 1-(3-Allyl-4-oxo-thiazolidin-2-yliden)-4-methyl-thiosemicarbazonsäure-allylester oder ein Salz davon, enthaltend, mit folgender Zusammensetzung:
- Wirkstoff: 1,0 g
- Carbopol 934 P: 1,0 g
- Glycerin: 3,0 g
- Isopropanol: 25,0 g
- Softigen® 767: 0,2g
- Wasser entmin. q.s. ad: 100,0 yg

### Beispiel 21:

In analoger Weise wie in den vorstehenden Beispielen 14 bis 20 beschrieben kann man auch pharmazeutische Präparate, enthaltend eine andere Verbindung der Formel I oder ein pharmazeutisch verwendbares Salz davon herstellen.

## Patentansprüche

1. Verbindungen der Formel I 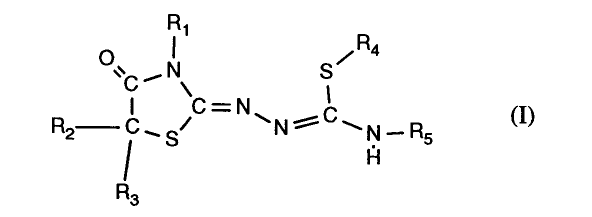 worin R₁ und R₅ Niederalkyl, Niederalk-2-en-1-yl oder auch Niederalk-2-in-1-yl bedeuten,
R₂ und R₃ unabhängig voneinander Wasserstoff oder Niederalkyl bedeuten oder gemeinsam für Niederalkyliden stehen und
R₄ Niederalkyl, Niederalkoxy, Niederalk-2-en-1-yl, Niederalk-2-in-1-yl, Arylniederalkyl, Arylniederalkenyl, oder auch Niederalkoxycarbonylniederalkyl bedeutet und ihre Salze und tautomeren Verbindungen und Doppelbindungsisomeren.

2. Verbindungen gemäss Anspruch 1 der Formel I, worin R₁ und R₅ C₁-C₄-Alkyl, C₃-C₅-Alk-2-en-1-yl oder C₃-C₅-Alk-2-in-1-yl bedeuten,
R₂ und R₃ unabhängig voneinander Wasserstoff oder C₁-C₄-Alkyl bedeuten oder gemeinsam C₁-C₄-Alkyliden darstellen und R₄ C₁-C₄-Alkyl, C₁-C₂-Alkoxy, C₃-C₅-Alk-2-en-1-yl, C₃-C₅-Alk-2-in-1-yl, Phenyl-C₁-C₂-alkyl bedeutet, und ihre Salze und tautomeren Verbindungen und Doppelbindungsisomeren.

3. Verbindungen gemäss Anspruch 1 der Formel I, worin der Rest R₁ C₃-C₅-Alk-2-en-1-yl, wie Allyl oder Methallyl oder C₃-C₅-Alk-2-in-1-yl, wie Prop-2-in-1-yl bedeutet, R₂ und R₃ beide Wasserstoff oder gleiche C₁-C₄-Alkylgruppen, wie Methylgruppen bedeuten oder gemeinsam C₁-C₄-Alkyliden, wie Methylen darstellen, R₄ C₁-C₄-Alkyl, wie Methyl oder Ethyl, C₁-C₂-Alkoxy, wie Methoxy oder Ethoxy, C₃-C₅-Alk-2-en-1-yl, wie Allyl, Methallyl oder Dimethylallyl, C₃-C₅-Alk-2-in-1-yl, wie Prop-2-in-1-yl oder Phenyl-C₁-C₂-alkyl, wie Benzyl bedeutet und R₅ C₃-C₅-Alk-2-en-1-yl, wie Allyl oder Methallyl oder C₁-C₄-Alkyl, wie Methyl oder Ethyl darstellt und ihre pharmazeutisch verwendbaren Salze und tautomeren Verbindungen und Doppelbindungsisomeren.

4. 1-(3-Allyl-4-oxo-thiazolidin-2-yliden)-4-methylthiosemicarbazonsäure-allylester oder ein pharmazeutisch verwendbares Salz davon.

5. 1-(3-Allyl-4-oxo-thiazolin-2-yliden)-4-methyl-thiosemicarbazonsäure-dimethylallyl-ester oder ein pharmazeutisch verwendbares Salz davon.

6. 1-(5,5-Dimethyl-3-methallyl-4-oxo-thiazolidin-2-yliden)-4-allyl-, thiosemicarbazonsäure-allylester oder ein pharmazeutisch verwndbares Salz davon.

7. 1-(5,5-Dimethyl-3-methallyl-4-oxo-thiazolidin-2-yliden)-4-alkyl-thiosemicarbazon-säure-alkylester oder ein pharmazeutisch verwendbares Salz davon.

8. 1-(3-Allyl-4-oxo-thiazolidin-2-yliden)-4-methyl-thiosemicarbazonsäure-benzylester oder ein pharmazeutisch verwendbares Salz davon.

9. 1-(3-Allyl-4-oxo-thiazolidin-2-yliden)-4-methyl-thiosemicarbazonsäuremethylester oder ein pharmazeutisch verwendbares Salz davon.

10. 1-(3-Allyl-4-oxo-thiazolidin-2-yliden)-4-methyl-thiosemicarbazonsäure-2-phenoxy-ethylester oder ein pharmazeutisch verwendbares Salz davon.

11. 1-(3-Allyl-4-oxo-thiazolidin-2-yliden)-4-methyl-thiosemicarbazonsäure-styrolester oder ein pharmazeutisch verwendbares Salz davon.

12. 1-(3-Allyl-4-oxo-thiazolidin-2-yliden)-4-methyl-thiosemicarbazonsäure-3-(2,4-dihydroxy-3-propyl-acetophenon-4-yl)-propylester oder ein pharmazeutisch verwendbares Salz davon.

13. 1-(3-Allyl-4-oxo-thiazolidin-2-yliden)-4-methyl-thiosemicarbazonsäure-2-hydroxy-ethylesters oder ein pharmazeutisch verwendbares Salz davon.

14. 1-(3-Allyl-4-oxo-thiazolidin-2-yliden)-4-methyl-thiosemicarbazonsäure-4-fluorobenzylester oder ein pharmazeutisch verwendbares Salz davon.

15. 1-(3-Allyl-4-oxo-thiazolidin-2-yliden)-4-methyl-thiosemicarbazonsäure-2-cyanobenzylester oder ein pharmazeutisch verwendbares Salz davon.

16. 1-(3-Allyl-4-oxo-thiazolidin-2-yliden)-4-methyl-thiosemicarbazonsäure-1-propargylester oder ein pharmazeutisch verwendbares Salz davon.

17. Verbindungen gemäss einem der Ansprüche 1 bis 16 zur Anwendung in einem Verfahren zur therapeutischen Behandlung des menschlichen oder tierischen Körpers.

18. Pharmazeutische Präparate, neben üblichen pharmazeutischen Hilfsstoffen als pharmazeutischer Wirkstoff enthaltend eine Verbindung gemäss einem der Ansprüche 1 bis 16 in freier Form oder in pharmazeutisch verwendbarer Salzform.

19. Verfahren zur Herstellung neuer Verbindungen der Formel I gemäss Anspruch 1, dadurch gekennzeichnet, dass man eine Verbindung der Formel II 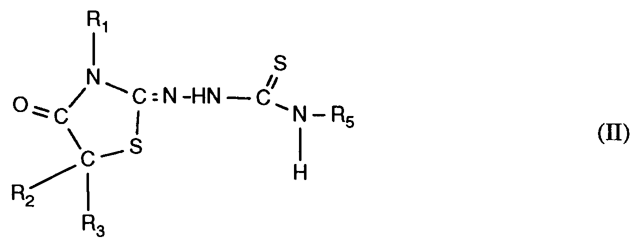 mit einer Verbindung der Formel III
R₄-X (III)
in der X eine nukleofuge Abgangsgruppe ist und R₁,R₂,R₃,R₄ und R₅ die oben angegebenen Bedeutungen haben, umsetzt, und gewünschtenfalls ein erfindungsgemäss erhältliches Isomerengemisch in die individuellen Isomeren auftrennt und das gewünschte Isomere isoliert und/oder eine erfindungsgemäss erhältliche freie Verbindung in ein Salz oder ein erfindungsgemäss erhältliches Salz in die freie Verbindung oder in ein anderes Salz überführt.

20. Das Verfahren der Beispiele, verfahrensgemäss verwendete neue Ausgangsstoffe, verfahrensgemäss gebildete neue Zwischenprodukte und verfahrensgemäss erhältliche neue Endstoffe.
